# EUROPEAN PATENT APPLICATION

(11) **EP 4 653 542 A1**
(43) Date of publication of application: **26.11.2025**
(21) Application number: 24177155.9
(22) Date of filing: 21.05.2024
(51) Int. Cl.: C12Q 1/6818, C12Q 1/6804

(54) **LABEL, MARKER AND METHOD FOR ANALYSING A BIOLOGICAL SAMPLE**

(71) Applicant: Leica Microsystems CMS GmbH, 35578 Wetzlar (DE); ATTO-TEC Gmbh, 57074 Siegen (DE)
(72) Inventor: Alsheimer, Soeren, 35578 Wetzlar (DE); Zilles, Alexander, 57074 Siegen (DE); Hoffmann, Jan-Erik, 57074 Siegen (DE)
(74) Representative: Schaumburg und Partner Patentanwälte mbB

(57) **Abstract**

A label (100, 200, 500, 702, 802, 900, 901, 1000, 1200, 1300, 1600) for analysing a biological sample is provided. The label (100, 200, 500, 702, 802, 900, 901, 1000, 1200, 1300, 1600) comprises a nucleic acid backbone (102, 502, 1002, 1102, 1202, 1301) and a first plurality of labelling moieties (104). The nucleic acid backbone (102, 502, 1002, 1102, 1202, 1301) has a duplex structure comprising at least 50% of nucleotides of the nucleic acid backbone (102, 502, 1002, 1102, 1202, 1301). In further aspects a marker (700, 800) comprising the label and a method for analysing biological samples is provided.

## Description

### Technical field

The invention relates to a label for analysing a biological sample. In further aspects, a marker comprising the label and a method for analysing a biological sample with the marker are provided.

### Background

The use of markers with labels that comprise a large number of individual dyes finds frequent application in fields like molecular biology and diagnostics, offering unparalleled sensitivity and specificity in detecting biological entities and chemical processes, in particular with fluorescence microscopy. These markers enable the visualization of complex cellular structures, tracking of molecular interactions, and high-throughput assays with high resolution. In particular, labels with a large number of dyes may result in bright labels.

However, the incorporation of numerous individual dyes within markers introduces a set of challenges, most notably self-quenching. Self-quenching occurs when the proximity of dyes within a marker leads to non-radiative energy transfer among them, significantly diminishing the emission efficiency and the fluorescence intensity that can be detected. This reduction in signal not only affects the sensitivity of measurements but also complicates quantitative analysis, making it difficult to accurately determine the concentration of targets or the efficiency of binding events. Self-quenching further poses a considerable challenge in high-resolution studies.

### Summary

It is an object to provide a label and a marker that are bright and have efficient emission characteristics.

The aforementioned object is achieved by the subject-matter of the independent claims. Advantageous embodiments are defined in the dependent claims and the following description.

In a first aspect, a label for analysing a biological sample is provided. The label comprises a nucleic acid backbone and a first plurality of labelling moieties. The nucleic acid backbone has a duplex structure comprising at least 50% of nucleotides of the nucleic acid backbone.

Preferably, the nucleic acid backbone is configured to allow formation of a duplex structure. As an example, the nucleic acid backbone may comprise a naturally occurring nucleic acid such as DNA or RNA.

Alternatively or in addition, the nucleic acid backbone may comprise or essentially consist of nucleic acid analogues. Generally, nucleic acid analogues are compounds which are structurally similar to naturally occurring RNA and DNA. Nucleic acids are chains of nucleotides, which are composed of three parts: a phosphate backbone, a pentose sugar, either ribose or deoxyribose, and one of four nucleobases. An analogue may have any of these parts altered. Thus, a nucleic acid analogue may be a non-naturally occurring, modified, artificial, or xeno nucleic acid (XNA), in particular. Whereas natural nucleic acids or naturally occurring nucleic acids (DNA and RNA) are generally sensitive to natural degradation agents such as nucleases, nucleic acid analogues are generally resistant to these degradation agents such as nucleases. Thus, this enables providing a nucleic acid backbone or label resistant to degradation by natural degradation agents, such as nucleases, and therefore a particular robust label.

More specifically, nucleic acid analogues may comprise modified nucleobases or may comprise partially or entirely a xeno nucleic acid (XNA) like for example PT-DNA, L-DNA, LNA, morpholino, peptide nucleic acid (PNA). The use of XNA may help to improve nuclease-resistance, which is desired in some applications.

The label enables bright emission of the plurality of labelling moieties, in particular with native optical properties of the labelling moieties, at a high efficiency. The duplex structure of the nucleic acid backbone provides a rigid or stiff backbone to attach the labelling moieties to. The rigidity or stiffness enables keeping the labelling moieties in place relative to each other and avoiding an aggregation of the labelling moieties, for example due to an attractive force between labelling moieties. Aggregation of labelling moieties may cause a change of their native optical properties such as their emission intensity and/or absorbance spectra. This makes unambiguous identification of the labelling moieties difficult or impossible.

A measure of the degree of rigidity or stiffness of a nucleic acid backbone, in particular a duplex structure is persistence length (L_{P}). The persistence length is a mechanical parameter quantifying polymer rigidity: the higher the persistence length, the more rigid the polymer. In the presence of monovalent or divalent salts, a nucleic acid duplex structure regularly has a persistence length of 30 to 55 nm, while single stranded nucleic acid is much more flexible, with a persistence length of 1.5 to 3 nm.

Thus, the nucleic acid backbone of the label may preferably have a persistence length in a range of 30 to 55 nm. This enables generating a rigid nucleic acid backbone as a stable framework for attaching labelling moieties. The rigidity of the nucleic acid backbone further enables bright fluorescent emission of the attached labelling moieties with their native optical properties.

The persistence length may be measured or determined using various methods including but not limited to single-molecule stretching techniques (e.g. optical, magnetic tweezers, dynamic force spectroscopy), FRET, dynamical mean-field theory and fluorescence correlation spectroscopy, atomic force microscopy as described in Roth et al. Nano Lett. 2018, 18, 11, 6703-6709, who used DNA-origami rods connected by a stretch of ssDNA of varying length and AFM to measure the persistence length in a way that allows the impact of secondary structures to be evaluated as well.

Qingjia Chi, Guixue Wang and Jiahuan Jiang (The persistence length and length per base of single-stranded DNA obtained from fluorescence correlation spectroscopy measurements using mean field theory; in Physica A: Statistical Mechanics and its Applications, Volume 392, Issue 5, 2013, Pages 1072-1079) discuss persistence length in relation to nucleic acids.

The labelling moieties, particularly of the first plurality of labelling moieties, are preferably optically detectable. For example, the labelling moieties may be fluorescent. Thus, the labelling moieties may be fluorophores, which may be optically detectable e.g. with a fluorescence microscope. Preferably, the first plurality of labelling moieties comprises between 2 and 200 labelling moieties, more preferably between 10 and 150 labelling moieties. In particular, each labelling moiety is attached to the nucleic acid backbone, preferably covalently attached. Alternatively, each labelling moiety may be attached to the nucleic acid backbone by a high affinity interaction such as a biotin-streptavidin linker.

In particular, the duplex structure is based on complementary base-pairing, for example, of at least one continuous sequence of nucleotides of the nucleic acid backbone. The duplex structure is preferably a double stranded segment of the nucleic acid backbone. For example, the duplex structure may be a secondary or tertiary structure, such as a double helix. In a particular example, the nucleic acid backbone may comprise several discontinuous double stranded segments that are the duplex structure of the nucleic acid backbone. In particular, at least 50% of nucleotides of the backbone may be paired to each other to form the duplex structure. In order of increasing preference, the duplex structure preferably comprises at least 60%, 70%, 80% or 90% of nucleotides of the nucleic acid backbone.

Preferably, a brightness of the label, in particular of the labelling moieties, in the presence of the duplex structure is at least 10% higher than a label without the duplex structure.

Preferably, adjacent labelling moieties of the first plurality of labelling moieties are attached to the nucleic acid backbone at a distance from each other between 1 to 50 nm. In a particularly preferred embodiment, adjacent labelling moieties of the first plurality of labelling moieties are attached to the nucleic acid backbone at a distance from each other of 5 to 40 nucleotides corresponding to about 1 to 13.2 nm, or 8 to 20 nucleotides corresponding to about 2.6 to 6.7nm. This enables dense spacing of the labelling moieties whilst avoiding electronic interactions between labelling moieties.

In some embodiments, where a label comprises a first and second labelling moiety such as fluorescent dyes of a FRET pair, it may be desirable to have a very dense packing of a large number of FRET donors that function like an antenna to collect excitation light and funnel the excitation to a smaller number, or may be even just one, FRET acceptor.

In some other embodiments multiple labels collective comprising a large number of FRET donors, such as 10, 50, 100, 1,000 FRET donors, being arranged using DNA nanotechnology to collect light and transfer energy to a small number of or a single FRET acceptor.

Preferably, the nucleic acid backbone comprises a main oligonucleotide with at least a first nucleotide sequence, wherein the nucleic acid backbone comprises at least a second nucleotide sequence, and wherein at least the first nucleotide sequence and the second nucleotide sequence form the duplex structure of the nucleic acid backbone. This enables an efficient generation of the duplex structure. In particular, complementary base-pairing between the first and second sequence generates the duplex structure. Thus, the first and second sequence are preferably complementary to each other and the first and second sequence form the duplex structure when they are hybridised with each other.

Preferably, the main oligonucleotide further comprises the second nucleotide sequence, and wherein the second nucleotide sequence is a reverse complement of the first nucleotide sequence. This enables generating the nucleic acid backbone from a single oligonucleotide and, in particular, generating a robust label. Thus, the complementary first and second nucleotide sequences form intramolecular base-pairs. For example, the nucleic acid backbone may form a stem-loop or hairpin loop, wherein the stem forms part of the duplex structure.

Preferably, the nucleic acid backbone comprises several of the main oligonucleotide. This enables generating a bright label, for example, comprising a large number of labelling moieties. The several main oligonucleotides may be connected to each other, for example by means of complementary sequences on each main oligonucleotide, or by means of connecting oligonucleotides that are complementary sequences of each main oligonucleotide. For example, each of the several main oligonucleotides may form a stem-loop or hairpin loop. In particular, each of the several main oligonucleotides may comprise a first and second nucleotide sequence that are unique to the particular one of the several main oligonucleotides. Thus, the first and the second nucleotide sequence of one of the several main oligonucleotides may only hybridise to each other and not to other first or second nucleotide sequences of other main oligonucleotides.

Preferably, the nucleic acid backbone comprises at least one auxiliary oligonucleotide comprising the second nucleotide sequence. This enables flexible generation of the duplex structure of the nucleic acid backbone of the label by addition of the at least one auxiliary oligonucleotide. Thus, in contrast to the embodiment where the first and second sequences are comprised by the main oligonucleotide, the first and second sequences may be on separate oligonucleotides. Thus, the main and auxiliary oligonucleotides may be separate oligonucleotides. In case the nucleic acid backbone comprises several auxiliary oligonucleotides, the main oligonucleotide may comprise several first nucleotide sequences and each auxiliary oligonucleotide may comprise the second nucleotide sequence. In particular, each second nucleotide sequence may be unique to a particular one of the auxiliary oligonucleotides and therefore hybridise to a particular one of the complementary first nucleotide sequences.

Preferably, the main oligonucleotide and the at least one auxiliary oligonucleotide are cross-linked with each other. This enables a particularly robust label. Further, the cross-link is preferably a covalent bond, for example comprising a triazole compound. The cross-linkage may be generated by click chemistry, such as copper-catalysed azide-alkyne cycloaddition. For example, the main oligonucleotide and/or the at least one auxiliary oligonucleotide may comprise a first reaction moiety and a second reaction moiety, respectively. When generating the label, initially the oligonucleotides of the nucleic acid backbone may be hybridised based on the complementary first and second sequences, subsequently, the oligonucleotides may be cross-linked in order to stabilise the label. Generally, the cross-linkages increase the rigidity or stiffness of the nucleic acid backbone, in particular of the duplex structure.

Alternatively or in addition, in case the nucleic acid backbone comprises several auxiliary oligonucleotides, the several auxiliary oligonucleotides may preferably be cross-linked with each other. In particular, each auxiliary oligonucleotide may be cross-linked with auxiliary oligonucleotides that are adjacent along the complementary main oligonucleotide.

Alternatively or in addition, the main oligonucleotide may preferably be cross-linked with itself. For example, a part of the main oligonucleotide towards a first end of the main oligonucleotide may be cross-linked to a part of the main oligonucleotide towards a second end of the main oligonucleotide. This enables generating a rigid circular label. In particular, the circular shape may generate ring tension, which significantly reduces conformational flexibility of the nucleic acid backbone.

Preferably, the nucleic acid backbone comprises a plurality of the first nucleotide sequence. This enables generating a label with a complex duplex structure. For example, the nucleic acid backbone may comprise a stem-loop formed based on one first and second nucleotide sequence and the nucleotide acid backbone may comprise another first nucleotide sequence for hybridising to an auxiliary oligonucleotide.

Preferably, the duplex structure, in particular, the first nucleotide sequence and the second nucleotide sequence, only comprises adenine and thymine nucleotides. This reduces quenching of labelling moieties, in particular, compared to the presence of guanine.

Preferably, the label comprises at least one nucleic acid binding molecule. This enables increasing the rigidity or stiffness of the nucleic acid backbone of the label. In particular, by aligning or ordering the nucleic acid backbone around the nucleic acid binding molecule. Examples of nucleic acid binding molecules include (eukaryotic) histones and bacterial DNA binding proteins, such as HU or integration host factor (IHF). A particular example of a bacterial DNA binding protein is Bd0055.

The nucleic acid binding molecule preferably binds to the duplex structure of the nucleic acid backbone. For example, the bacterial DNA binding protein Bd0055 binds duplex DNA on its outside surface and induces a rod formation in the duplex DNA. The binding of the nucleic acid binding molecules may be non-specific or sequence specific. In the latter case, the nucleic acid backbone, in particular the duplex structure, may comprise specific sequences configured to bind nucleic acid binding molecules.

Preferably, the labelling moieties are hydrophobic labelling moieties. The label, in particular its nucleic acid backbone, provides a rigid structure for hydrophobic labelling moieties. Generally, in an aqueous solution hydrophobic labelling moieties have a tendency to aggregate. In particular, hydrophobic labelling moieties that are arranged in close proximity on a flexible single stranded DNA backbone may lead to formation of aggregates. This aggregation changes the optical properties of these labelling moieties such as their absorption wavelength and/or in particular their fluorescence brightness, compared to non-aggregated hydrophobic labelling moieties. By providing the label with the hydrophobic labelling moieties, the nucleic acid backbone avoids the aggregation of the labelling moieties and therefore enables use of the hydrophobic labelling moieties at their native optical properties.

Examples of hydrophobic labelling moieties that are regularly used to analyse biological samples include ATTO 390, ATTO 425, ATTO 550, ATTO Rho12, ATTO 633, and ATTO 647N. In particular, the dyes mentioned above may exhibit undesired aggregation when multimerized on a single stranded DNA nucleic acid backbone with a dye-to-dye distance of 8 nt.

In contrast, hydrophilic labelling moieties may include ATTO 488, and ATTO 532, and ATTO 565 for example. In particular, the dyes mentioned above do not exhibit undesired aggregation when multimerized on a ssDNA nucleic acid backbone with a dye-to-dye distance of 8 nt.

Preferably, the labelling moieties, in particular of the first plurality of labelling moieties, are attached to the nucleic acid backbone at a distance from each other in a range from one to ten nucleotides. This enables generating compact labels. Preferably, the labelling moieties are attached to nucleic acid backbone such that the labelling moieties are oriented in the same direction. In this case, the labelling moieties may be attached to every tenth nucleotide, in particular, of one of the oligonucleotides of the nucleic acid backbone.

Preferably, the labelling moieties are attached to the duplex structure. By attaching the labelling moieties to the duplex structure, the labelling moieties are attached to the part of the nucleic acid backbone that has an increased rigidity. This enables generating labels with labelling moieties, wherein the labelling moieties have a consistent distance from each other, that is not substantially influenced by the flexibility of the nucleic acid backbone. This avoids undesired interactions between the labelling moieties. In particular, the labelling moieties may be attached to the first nucleotide sequence or the second nucleotide sequence.

Preferably, the auxiliary oligonucleotide comprises at least a second plurality of labelling moieties configured to excitonically interact, for example via a fluorescence resonance energy transfer, with the first plurality of labelling moieties, e.g. as described in WO 2023/198291 A1, the complete content thereof being incorporated by reference. This enables specifically influencing or modulating the optical properties of the first plurality of labelling moieties. In particular, this enables generating a large number of distinguishable labels.

Preferably, the label comprises a plurality of reactive oxygen species quenchers, wherein each of the reactive oxygen species quenchers is attached to the nucleic acid backbone in close proximity to a labelling moiety, in particular of the first plurality of labelling moieties. This enables an increase in the lifetime of the labelling moieties, in particular, by reducing the occurrence or concentration of reactive oxygen species in the proximity of the labelling moieties. This reduces the deleterious effects of reactive oxygen species on the labelling moieties. In particular, the close proximity of the reactive oxygen species quenchers to the labelling moieties may be chosen such that the quenching action of the quenchers has an advantageous effect on the labelling moieties. Preferably, the reactive oxygen species quenchers may be attached to the nucleic acid backbone at a distance in a range of 0.3 nm to 15 nm to one of the labelling moieties. Examples of reactive oxygen species quenchers include DABCO and lycopene. In a particular example, the labelling moieties may be attached to one of the main oligonucleotide and the auxiliary oligonucleotide and the reactive oxygen species quencher may be attached to the other one of the main oligonucleotide and the auxiliary oligonucleotide.

Preferably, the nucleic acid backbone comprises 20 to 200 nucleotides, more preferably 50 to 150 nucleotides. Alternatively or additionally, the main oligonucleotide has a length of 20 to 120 nucleotides, in particular 30 to 120 nucleotides. This enables generating a compact label.

Preferably, the nucleic acid backbone or the main oligonucleotide comprises a barcode sequence. The barcode sequence may be for hybridising the label to a complementary sequence of an affinity reagent, for example. This enables easily and specifically attaching the label to an affinity reagent. In particular, the barcode sequence may be comprised by the main oligonucleotide, for example as a single stranded free end of the main oligonucleotide.

Preferably, the label might comprise at least 5 labelling moieties. In particular, the labelling moieties may be covalently conjugated or attached to either the main oligonucleotide or to the auxiliary oligonucleotide.

Preferably, the nucleic acid backbone may comprise modified nucleobases or at least partially might comprise nucleic acid analogues.

In another aspect, a marker for analysing a biological sample is provided. The marker comprises a label, in particular as described above. The marker further comprises an affinity reagent configured to specifically bind to a target analyte. The affinity reagent may be an antibody, an antibody fragment, or an aptamer, in particular a nucleic acid based aptamer, for example. The affinity reagent enables specifically binding the label to a particular target analyte in the biological sample.

Preferably, the affinity reagent comprises a barcode sequence. The barcode sequence is for hybridising the affinity reagent to a complementary barcode sequence of the label, for example. This enables easily and specifically attaching the label to the affinity reagent. Alternatively, the label may be attached to the affinity reagent by means of a covalent linker.

In a further aspect, a method for analysing a biological sample is provided. The method comprises the steps of introducing into the biological sample at least one marker and/or parts of at least one marker, in particular as described above. The method further includes the step of generating a readout of the biological sample with the marker. This enables identifying and/or locating the target analyte in the biological sample that the marker binds to. In particular, the readout may be an optical readout, for example, generated by means of a microscope.

In particular, the step of introducing into the biological sample parts of at least one marker includes introducing at least an affinity reagent, a nucleic acid backbone and a first plurality of labelling moieties into the biological sample. The parts may be introduced either separately from each other in time and/or spatially, or together. Thus, the marker may either be generated prior to introduction into the biological sample or the marker may be generated from its parts within the biological sample. The latter may be advantageous in case the marker, in particular the label, is bulky and/or rigid compared to its individual parts and the biological sample is a tissue section, for example. In this case, the more compact and/or flexible individual parts may be introduced separately and the bulky and/or rigid marker is only generated in situ. In particular, the formation of the duplex structure of the nucleic acid backbone of the label in situ may be advantageous, since the main oligonucleotide as a single strand may more easily penetrate or diffuse into the biological sample compared to the duplex structure. Thus, preferably the duplex structure of the nucleic acid backbone of the label may be generated in situ, for example by adding the auxiliary oligonucleotide after adding the main oligonucleotide of the nucleic acid backbone of the label.

In addition, the cross-linking of the main oligonucleotide and/or the auxiliary oligonucleotide may be carried out after introducing the parts of the marker into the biological sample.

Preferably, prior to or after generating the readout and after the step of introducing the parts of at least one marker, an environmental parameter is changed in order to generate or ungenerate the duplex structure. This enables controlling the fluorescent emission of the label. For example, the duplex structure of the nucleic acid backbone of the label may be melted by an increase in temperature, in particular above the melting temperature of the duplex structure, causing the nucleic acid backbone to relax and the labelling moieties to aggregate. Aggregation of labelling moieties may cause a change in fluorescent emission due to self-quenching. An optical readout might be generated in this situation. The duplex structure may be generated by subsequently reducing the temperature, causing the nucleic acid backbone to stiffen and the labelling moieties to separate. Preferably, labels with nucleic acid backbones comprising between 10 to 40 nucleotides are used when the method comprises steps of environmental parameters changes.

Generally, a plurality of markers may be introduced into the biological sample, the markers being specific to respective target analytes, in order to identify a large number of (different or the same) target analytes at the same time. Preferably, a target analyte is identified and/or localised within the biological sample based on the label(s) of the marker(s), in particular the labelling moieties, associated with the target analyte in the optical readout. Markers may be physically constituted before introducing them into the biological sample. Alternatively, affinity reagents may be barcoded with oligonucleotide barcodes and introduced into the biological sample initially and the labels comprising complementary barcode oligonucleotides may be introduced into the biological sample in a subsequent step and may then attach to their respective affinity reagent thereby forming the respective markers within the biological sample.

The marker and the method have the same advantages as the label. Further, the marker and the method may be supplemented with the features of the label described in this document, in particular, the features of the dependent claims of the label.

### Short Description of the Figures

Hereinafter, specific embodiments are described referring to the drawings, wherein:
- Figure 1: is a schematic view of a label comprising a nucleic acid backbone and a plurality of labelling moieties,
- Figure 2: is a schematic view of a label without an auxiliary oligonucleotide,
- Figure 3A: shows graphs of absorbance spectra for different numbers of labelling moieties,
- Figure 3B: shows graphs of the increase in absorbance relative to the number of labelling moieties,
- Figure 3C: shows graphs of the increase in emission relative to the number of labelling moieties,
- Figure 4A: shows graphs of the increase in emission for labels,
- Figure 4B: shows graphs of the increase in emission for labels over time,
- Figure 4C: shows a graph of a kinetic of an experiment,
- Figure 5: is a schematic view of a label according to a second embodiment,
- Figure 6: is a schematic view of the label according to Fig. 5,
- Figure 7: is a schematic view of a marker comprising a hairpin loop label,
- Figure 8: is a schematic view a marker comprising a hairpin loop label according to a second embodiment,
- Figure 9: is a schematic view of circular labels,
- Figure 10: is a schematic view of a label according to a third embodiment,
- Figure 11: is a schematic view of a label according to a fourth embodiment,
- Figure 12: is a schematic view of a label according to a fifth embodiment and steps to generate the label,
- Figure 13: is a schematic view of a label according to a sixth embodiment and steps to generate the label,
- Figure 14: is a schematic view of the label according to Fig. 1 and a complex of the label with a nucleic acid binding molecule,
- Figure 15: is a schematic view of the label according to Fig. 1 and a complex of the label with a further nucleic acid binding molecule, and
- Figure 16: is a schematic view of steps to control the optical properties of a label.

### Detailed Description

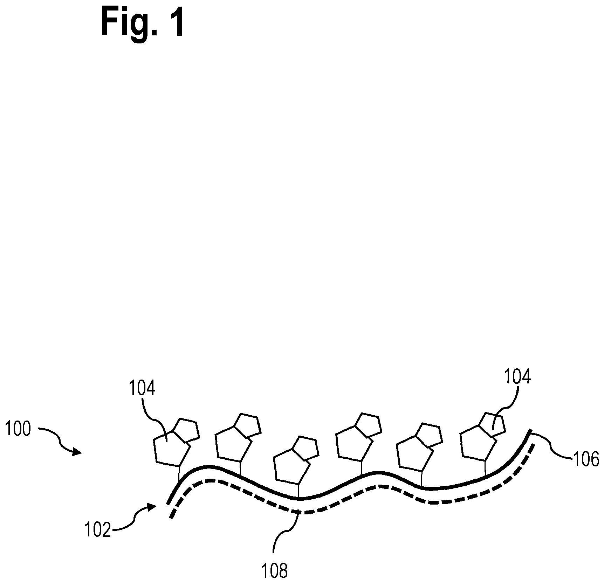

Figure 1 is a schematic view of a label 100 comprising a nucleic acid backbone 102 and a plurality of labelling moieties 104. The nucleic acid backbone 102 comprises a main oligonucleotide 106 and an auxiliary oligonucleotide 108. The labelling moieties 104 are attached to the main oligonucleotide 106, in particular covalently.

The main oligonucleotide 106 has a first nucleotide sequence and the auxiliary oligonucleotide 108 has a second nucleotide sequence. The first nucleotide sequence and the second nucleotide sequence are complementary to each other. Thus, the main oligonucleotide 106 and the auxiliary oligonucleotide 108 hybridise to each other along their entire length. This results in the nucleic acid backbone 102 having a duplex structure generated when the main oligonucleotide 106 and the auxiliary oligonucleotide 108 hybridise to each other. Thus, the main oligonucleotide 106 and the auxiliary oligonucleotide 108 form a double stranded nucleic acid molecule. In particular, the duplex structure of the nucleic acid backbone 102 may form a double helix from the main oligonucleotide 106 and the auxiliary oligonucleotide 108. Preferably, the nucleic acid backbone 102 comprises 20 to 200 nucleotides.

A corresponding marker may comprise the label 100 attached to an affinity reagent (not shown), such as an antibody, antibody fragment, aptamer, affibody, artificial polymeric binder, nanobody, or oligonucleotide probes. By means of the affinity reagent, the label 100 may specifically bind to a target analyte in a biological sample.

The labelling moieties 104 may be fluorophores like fluorescent proteins, or organic fluorescent dyes, QDots, Pdots, polymer dyes, SMILEs. The labelling moieties 104 may be for example fluorescent dyes such as ATTO 390, ATTO 425, ATTO 430, ATTO 465, ATTO 488, ATTO 532, ATOT 542, ATTO Rho3B, ATTO 565, ATTO 594, ATTO 643, ATTO 647N, ATTO 680, ATTO 700, ATTO 740, BODIPY dyes, AlexFluor^{™} dyes, Cy dyes, CF dyes. Such fluorescent dyes may be derivatives of a variety of base chromophore structures including but not limited to acridine, anthracenes, arylmethine, dipyrromethene, fluorescein, cyanines, coumarines, pyrene, oxazines, rhodamines, carborhodamines, squaraine, squaraine rotaxane, tetrapyrrole, triangelium dyes, xanthenes. Further labelling moieties 104 may be fluorescent proteins like GFP, mCherry, EYFP, Phycoerythrin, Alllophycocyanin or non-protein organic fluorophores like the aforementioned dyes or polymer dyes like SuperNova vn428 for example. Further, the labelling moieties 104 may be fluorescence quenchers like ATTO 540Q, ATTO 575Q, ATTO 580Q, ATTO 612Q. Further, the labelling moieties 104 may comprise fluorescent dyes that fluoresce only under a first condition but are essentially non-fluorescent under a second condition like for example ATTO MB2. Further, the labelling moieties 104 in the sense of this document may be a tandem dye, i.e. to covalently connected fluorophores.

The label 100, in particular its labelling moieties 104, and the marker comprising the label 100 may be optically detected, for example, by means of a microscope.

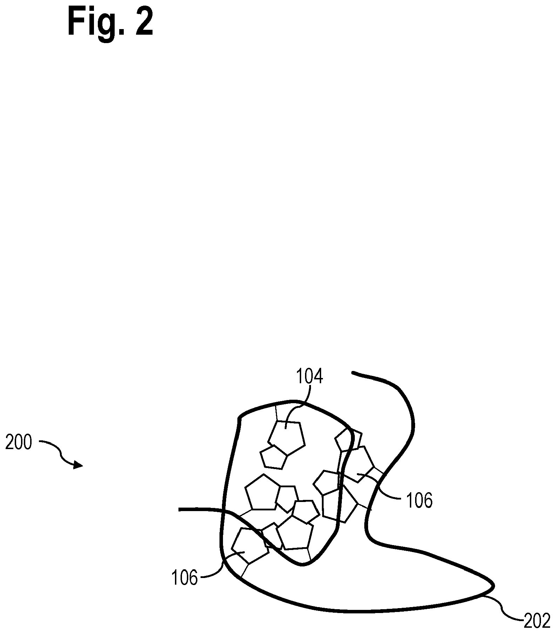

Figure 2 is a schematic view of a label 200 without the auxiliary oligonucleotide 108, in particular, without the second nucleotide sequence. In comparison to the label 100, the label 200 has a substantially more flexible nucleic acid backbone 202, consisting of one single stranded oligonucleotide.

The more flexible nucleic acid backbone 202 may result in the labelling moieties 104 aggregating, in particular when the labelling moieties 104 are hydrophobic and in an aqueous solution. This may result in a change in the optical properties of the labelling moieties 104, such as their absorbance wavelength or intensity. In particular, the labelling moieties 104 may shift their absorbance wavelength and/or exhibit self-quenching. This change in optical properties may complicate or prevent identification of the label 200 in a biological sample.

In contrast, the duplex structure of the nucleic acid backbone 102 enables the nucleic acid backbone 102 to be of significantly higher rigidity or stiffness. This results in the label 100 being substantially rod-shaped compared to the aggregated nature of the label 200. Moreover, this prevents the labelling moieties 104 attached to the nucleic acid backbone 102 from aggregating. In absence of aggregation, the labelling moieties 104 do not change their optical properties or exhibit their initial or inherent optical properties.

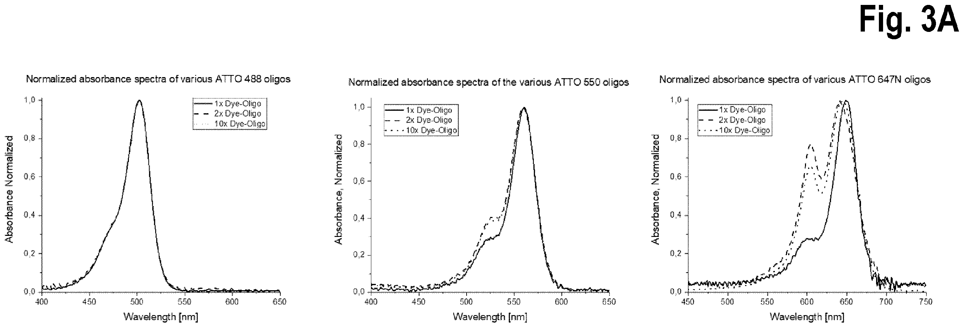

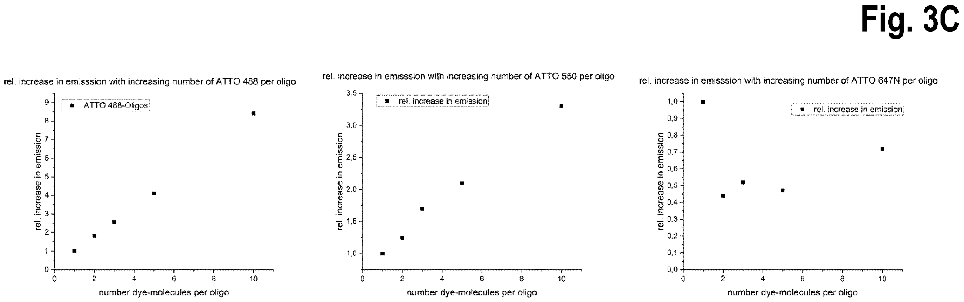

Figure 3A to 3C are views of graphs showing the effect of aggregating labelling moieties attached to oligonucleotides of varying length ranging from ~20 nt for 1x dye-conjugated oligo to ~120 nt 10x dye-conjugated oligo. The dye-to-dye distance is 8 nt in these examples. Figure 3A-3C show that the multimerization of hydrophilic dyes like ATTO 488 on a ssDNA nucleic acid backbone, i.e. an oligonucleotide, is a viable strategy to generate bright and compact DNA-based fluorescent labels. For dyes such as ATTO 550, which is moderately hydrophobic, or even more hydrophobic dyes such as ATTO647N, the multimerization of dyes on a ssDNA backbone leads to aggregation, which is apparent from the absorption spectra (Figure 3A, i.e. second peak) and strong drop in fluorescence emission (Figure 3C). It is concluded that the flexibility of the ssDNA nucleic acid backbone is so high (in other words the persistence length is short) that the dye molecules can practically freely aggregate, despite the fact that they are connected to the polymeric backbone. It is proposed that a reduction of the flexibility or in other words an increase in persistence length or rigidity of the nucleic acid backbone, may reduce or abolish aggregation and bring back the desired increase in label brightness with an increase in the number of dyes.

Such an aggregation is shown schematically in Fig. 2.

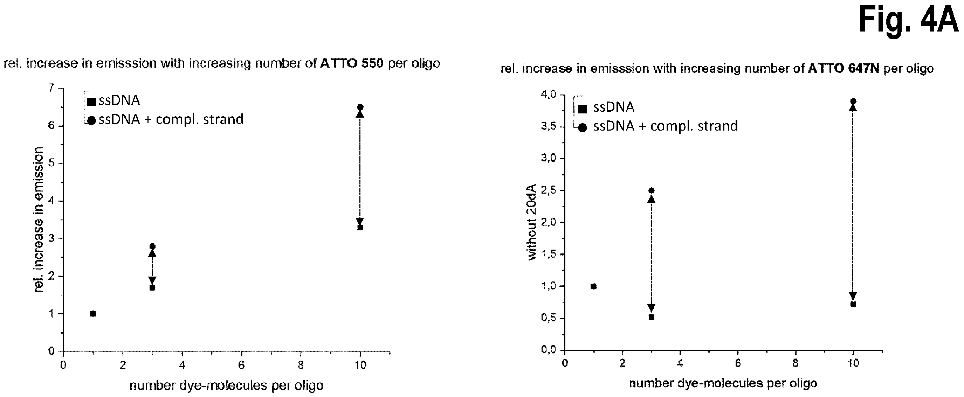

In fact, experiments showed that the ssDNA nucleic backbone of the label was hybridized with one or more complementary oligonucleotide(s) to generate dsDNA nucleic acid backbones and a "rescue" of the ATTO 550 and ATTO 647N labels fluorescence emission by formation of a nucleic acid backbone comprising a duplex (Figure 4A). Duplex formation increases the rigidity or persistence length of the nucleic acid backbone to the point where the connected dye molecules can no longer freely aggregate. This is schematically depicted in Figure 1.

Figure 3A shows graphs of absorbance spectra for different numbers of labelling moieties ATTO 488, ATTO 550 and ATTO 647N attached to single stranded oligonucleotides. In particular, labelling moieties prone to aggregation such as ATTO 550 and ATTO 647N show a shift in their absorbance spectra when an increasing number of labelling moieties is attached to a single stranded oligonucleotide.

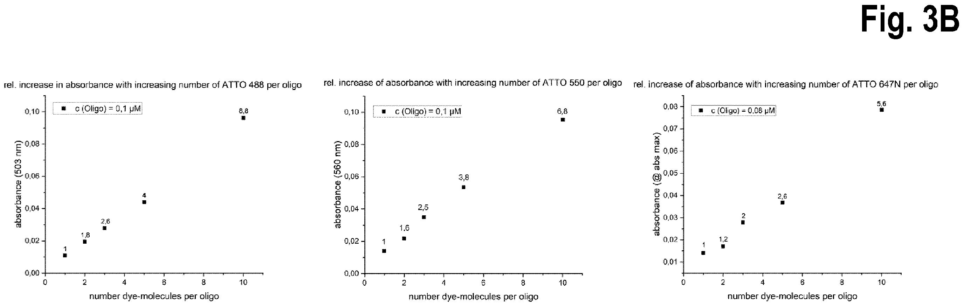

Figure 3B shows graphs of the increase in absorbance relative to the number of labelling moieties attached to a single stranded oligonucleotide. In particular, labelling moieties prone to aggregation such as ATTO 550 and ATTO 647N show non-proportional increase in absorbance with an increase in the number of labelling moieties.

Figure 3C shows graphs of the increase in emission relative to the number of labelling moieties attached to a single stranded oligonucleotide. In particular, labelling moieties prone to aggregation such as ATTO 550 and ATTO 647N show a reduced or no increase in emission with an increase in the number of labelling moieties.

Figure 4A to 4C are views of graphs showing the effect of labels having a duplex structure (for example according to Fig. 1) compared to labels having no duplex structure (for example according to Fig. 2).

Figure 4A shows graphs of the increase in emission for labels. Labels with no duplex structure (ssDNA) and aggregated labelling moieties similar to the Figs. 3A to 3C have a decrease in emission compared to labels with a duplex structure (ssDNA + compl. Strand).

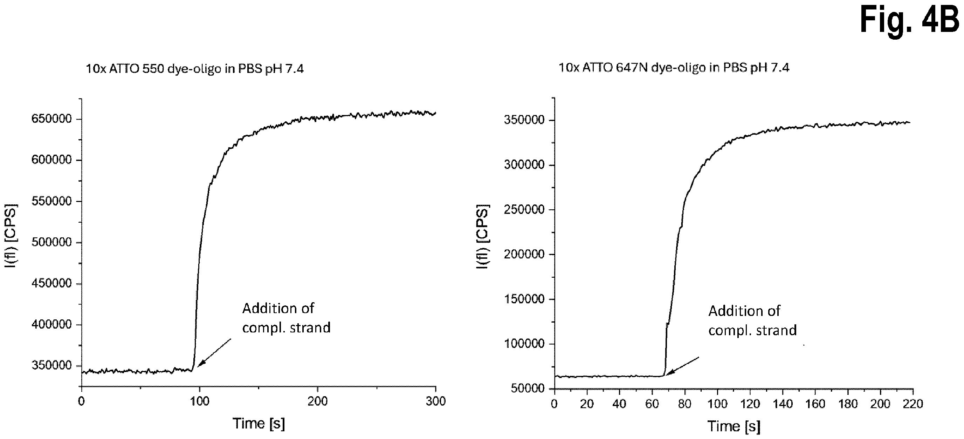

Figure 4B shows graphs of the increase in emission for labels over time. Only when the duplex structure is formed, for example after addition of an auxiliary oligonucleotide (compl. Strand), the emission intensity of the labelling moieties increases.

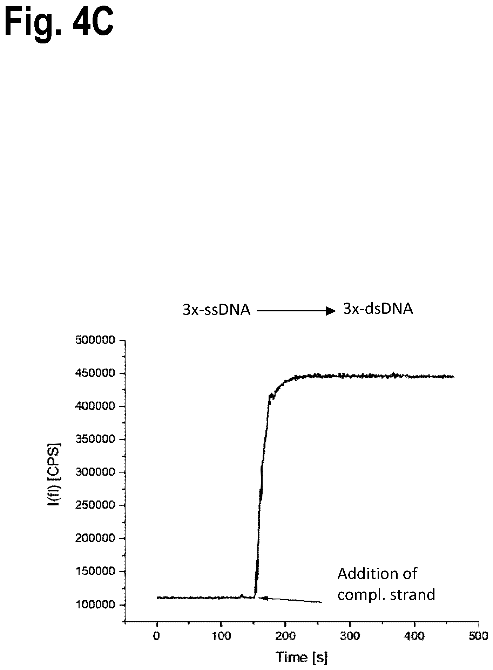

Figure 4C shows a kinetic of an experiment, in which fluorescence emission was measured. In this experiment the fluorescence emission intensity [I(fl)] in counts per second (CPS) of a label comprising 3 dye molecules of ATTO 647N with a ssDNA nucleic acid backbone ("3x-ssDNA") is followed over time. Following to the addition of complementary strand at around 150s the fluorescence emission sharply increases due to the formation of the duplex or dsDNA nucleic acid backbone of the label ("3x-dsDNA").

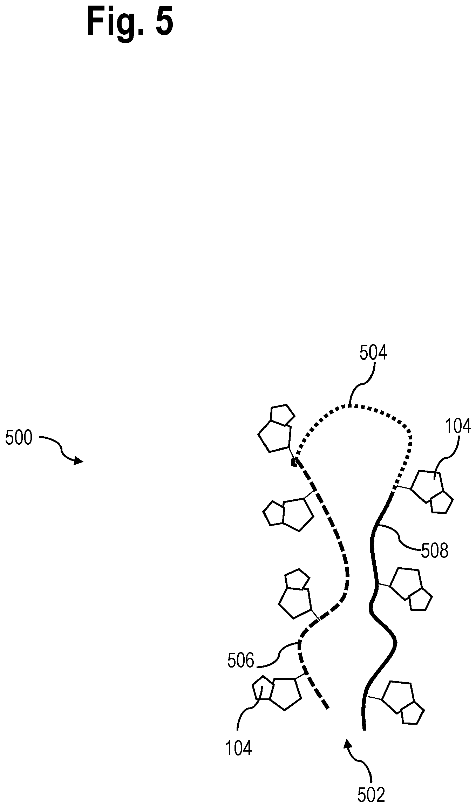

Figure 5 is a schematic view of a label 500. The label 500 comprises a plurality of the labelling moieties 104. The label 500 further comprises a nucleic acid backbone 502 consisting of a single main oligonucleotide 504 with a first nucleotide sequence 506 and a second nucleotide sequence 508. The second nucleotide sequence 508 is the reverse complement of the first nucleotide sequence 506. Thus, the first nucleotide sequence 506 and the second nucleotide sequence 508 may hybridise to form a duplex structure. In Fig. 5 the first and second nucleotide sequences 506, 508 are shown in a non-hybridised state without the duplex structure. The nucleic acid backbone 502 may form a stem-loop upon hybridisation of the first and second nucleotide sequences 506, 508. The resulting stem or duplex structure of the nucleic acid backbone 502, comprising the first and second nucleotide sequences 506, 508, may, in particular, form a double helix structure.

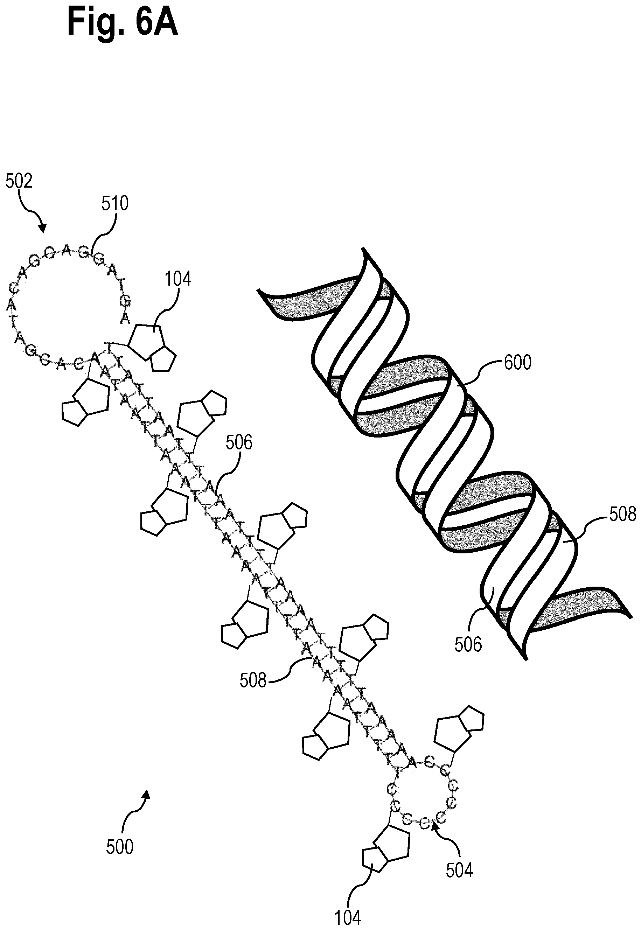

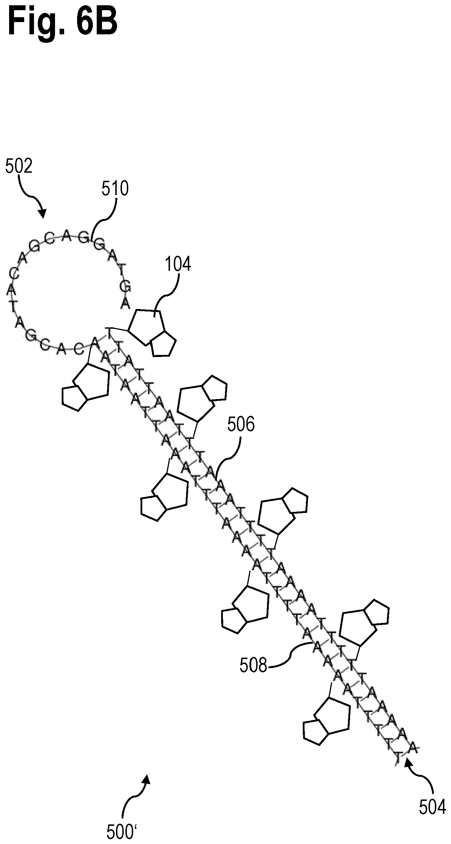

Figure 6A is a schematic view of the label 500 where the main oligonucleotide 504 is represented by individual letters of the respective nucleobases (adenine (A), cytosine (C), guanine (G), thymine (T)), as a specific example of the main oligonucleotide 504. As described for Fig. 5, the main oligonucleotide 504, in particular the first and second nucleotide sequences 506, 508, of the nucleic acid backbone 502 may form a double helix, which is illustrated in Fig. 6 by the structure indicated with reference sign 600. The label 500 further comprises a barcode 510 by way of example only.

Preferably, the labelling moieties 104 are attached to the duplex structure, in particular, to the first and second nucleotide sequences 506, 508, of the nucleic acid backbone 502.

Preferably, the first and second nucleotide sequences 506, 508 only comprise the nucleobases adenine and thymine. In particular, the first and second nucleotide sequences 506, 508 do not comprise the nucleobase guanine. This is due to guanine acting as a fluorescence quencher.

Figure 6B is a schematic view of the label 500' according to another embodiment. In this embodiment, as opposed to the embodiment shown in Figure 6A, the main oligonucleotide 504 only comprises the first and second nucleotide sequences 506, 508. The advantage is that the label 500' can be generated from two shorter nucleotide sequences, which increases synthesis yield and thereby reduces production costs. The label 500' further comprises the barcode 510 by way of example only.

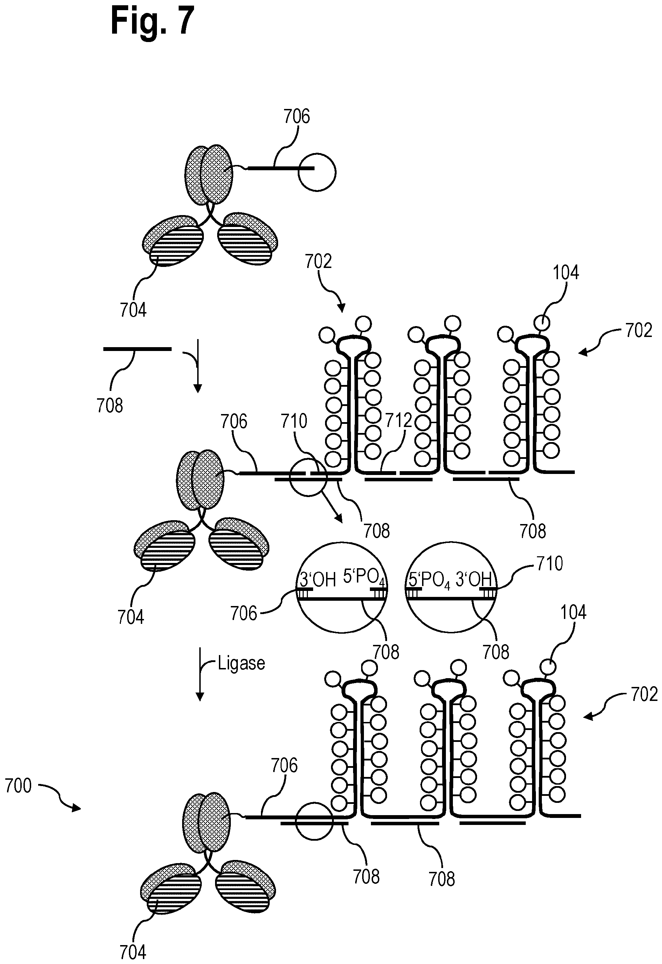

Figure 7 is a schematic view of a marker 700 comprising several hairpin loop labels 702, as well as steps to generate the marker 700. The marker 700 further comprises an affinity reagent 704 for specifically binding the marker 700 to a target analyte. The affinity reagent 704 may be an antibody, for example. Alternatively, the affinity reagent 704 may be an antibody fragment or an aptamer. The labels 702 may preferably have the features described for the label 500.

The marker 700 may further comprise a barcode oligonucleotide 706 that is attached to the affinity reagent 704 and by means of which the labels 702 may be attached to the affinity reagent 704.

In order to generate the marker 700 from the affinity reagent 704 and the labels 702 an overlapping complementary attachment oligonucleotide 708 may be introduced to the parts of the marker 700 (middle view of Fig. 7). In particular, the labels 702 may be attached to each other and attached to the affinity reagent 704 by means of the attachment oligonucleotide 708. The attachment oligonucleotide 708 is at least partially complementary to the barcode oligonucleotide 706 and to a free end 710 of a main oligonucleotide of a nucleic acid backbone of the label 702. The attachment oligonucleotide 708 may also be complementary to a second free end 712 of the main oligonucleotide of the label 702. This enables attaching the labels 702 to each other.

In an alternative embodiment, a plurality of attachment oligonucleotides 708 may be introduced to the parts of the marker 700. Further, the barcode oligonucleotide 706 and the free ends 710, 712 of each label 702 may have a unique sequence and at least one of the attachment oligonucleotides 708 may have a sequence complementary to the respective adjacent sequences of the barcode oligonucleotide 706 and free ends 710, 712. This enables specifically attaching the individual labels 702 to the marker 700.

In a subsequent step, the labels 702, in particular their nucleic acid backbones, may be ligated to each other and to the attachment oligonucleotide 706 (bottom view of Fig. 7) by adding a ligase. This catalyses the formation of a phosphodiester bond between the 3'-hydroxyl group (-OH) at one end of one of the nucleotide strands 706, 710, 712 and the 5'-phosphate group (-PO₄) of another end of the respective nucleotide strands 706, 710, 712. This results in the robust marker 700.

In an alternative embodiment the marker 700 may comprise only a single label 702.

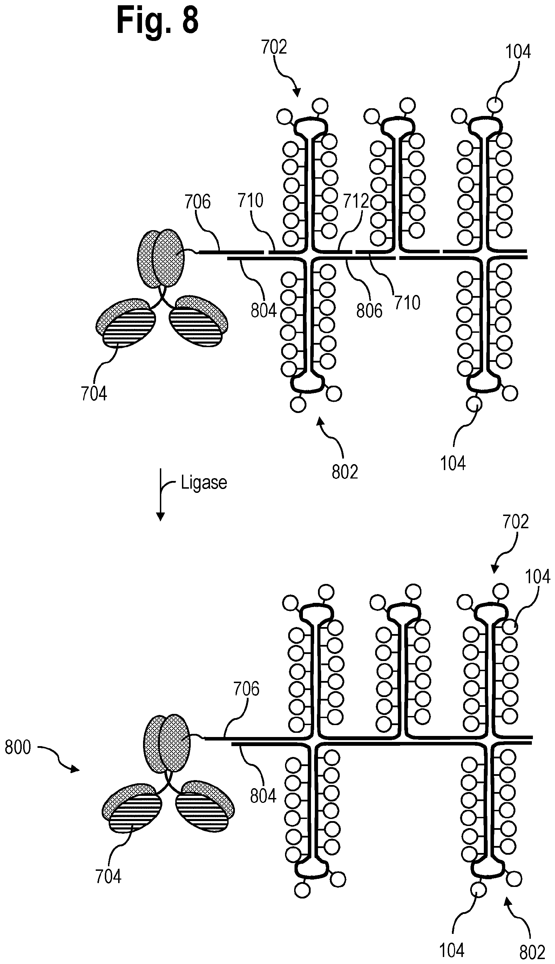

Figure 8 is a schematic view of a marker 800 comprising hairpin loop labels 702, 802, as well as a step to generate the marker 800. The marker 800 further comprises the affinity reagent 704 with the barcode oligonucleotide 706. The labels 802 may preferably have the features described for the label 500.

Similarly to the marker 700 described together with Fig. 7, the label 800 comprises the label 702. However, instead of attaching the label 702 to the barcode oligonucleotide 706 by means of the attachment oligonucleotide 708, the label 702 is attached to the barcode oligonucleotide 706 by means of the label 802 to generate the marker 800. In particular, the label 802 comprises a complementary barcode oligonucleotide 804, which is at least partially complementary to the barcode oligonucleotide 706 of the affinity reagent 704 and to the free end 710 of the label 702. The complementary barcode oligonucleotide 804 is preferably a single stranded part of the nucleic acid backbone of the label 802.

The label 802, in particular its main oligonucleotide, may have a further end 806, which is at least partially complementary to the free ends 710, 712 of adjacent labels 702. The parts 804, 806 of the label 802 may alternatively both be at least partially complementary to the free ends 710, 712 of respective adjacent labels 702 in order to connect a plurality of labels 702 to each other.

As described for marker 700, in a subsequent step, the adjacent labels 702, 802, in particular their nucleic acid backbones, may be ligated to the respective adjacent labels 702, 802 and to the attachment oligonucleotide 706 (bottom view of Fig. 8) by adding a ligase. This catalyses the formation of a phosphodiester bond between the 3'-hydroxyl group (-OH) at one end of one of the nucleotide strands 706, 710, 712, 804, 806 and the 5'-phosphate group (-PO₄) of another end of the respective nucleotide strands 706, 710, 712, 804, 806. This results in the robust marker 800.

In view of the duplex structure of the nucleic acid backbones of the labels 702, 802 and the duplex structure that is generated from hybridising the oligonucleotides 706, 710, 712 to the attachment oligonucleotide 708 or the oligonucleotides 804, 806, the markers 700, 800 have a rigid structure. This enables keeping the labelling moieties 702 in place relative to each other and avoiding their aggregation.

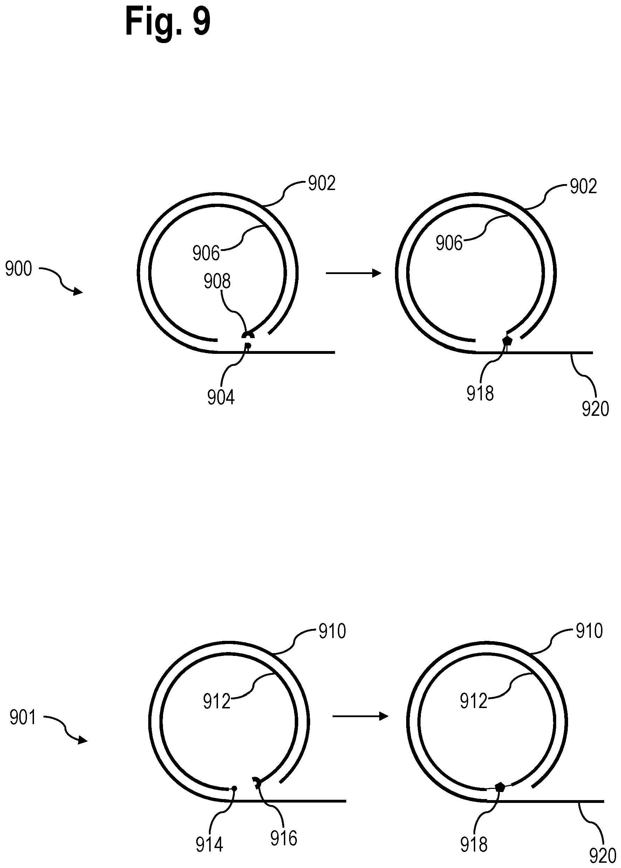

Figure 9 is a schematic view of circular labels 900, 901. The label 900 comprises a main oligonucleotide 902 with a first reaction moiety 904 and an auxiliary oligonucleotide 906 with a second reaction moiety 908. The main oligonucleotide 902 comprises a first nucleotide sequence that is complementary to a second nucleotide sequence of the auxiliary oligonucleotide 906. The main oligonucleotide 902 and the auxiliary oligonucleotide 906 may therefore hybridise and form a duplex structure, in particular a double helix. The first reaction moiety 904 and the second reaction moiety 908 are both arranged towards a 3' or a 5' end of the respective oligonucleotide 902, 906.

The label 901 similarly comprises a main oligonucleotide 910 and an auxiliary oligonucleotide 912, which comprise respective complementary first and second nucleotide sequences that may form a duplex structure. The auxiliary oligonucleotide 912 comprises a first reaction moiety 914 towards a first end, for example at a 3' or a 5' end, of the auxiliary oligonucleotide 912. In addition, the auxiliary oligonucleotide 912 comprises a second reaction moiety 916 towards a second end, for example a 5' or a 3' end, of the auxiliary oligonucleotide 912, opposing the first end.

The first reaction moieties 904, 914 and the second reaction moiety 908, 916 are configured to form a covalent bond 918 with each other, in particular under specific reaction conditions or in the presence of a particular catalyst. This cross-links the respective parts of the labels 900, 901. For example, the first reaction moieties 904, 914 and the second reaction moieties 908, 916 may be respective click chemistry groups, in particular based on copper-catalysed azide-alkyne cycloaddition.

The first reaction moieties 904, 914 and the second reaction moiety 908, 916 are preferably not attached along the first or second nucleotide sequence of the respective oligonucleotide 902, 906, 910, 912. Instead, the first reaction moieties 904, 914 and the second reaction moiety 908, 916 are preferably attached to the respective oligonucleotide 902, 906, 910, 912 next to the first or second nucleotide sequence.

The main oligonucleotides 902, 910 of the labels 900, 901 may preferably comprise a single stranded free end 920 that is complementary to a barcode oligonucleotide of an affinity reagent, in order to attach the labels 900, 901 to the affinity reagent. The free end 920 does not comprise the first nucleotide sequence of the main oligonucleotide 902, 910. In case of the label 900, the first reaction moiety 904 is preferably arranged at a distance from a 3' or 5' end of the main oligonucleotide 902 in order to generate the free end 920.

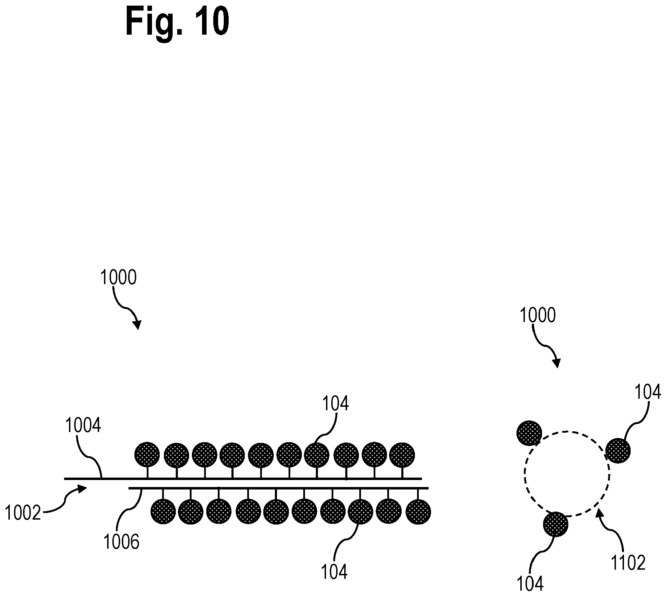

Figure 10 is a schematic view of a label 1000. The label 1000 comprises a nucleic acid backbone 1002 with a main oligonucleotide 1004 and an auxiliary oligonucleotide 1006. A plurality of labelling moieties 104 is attached to a duplex structure form between the main oligonucleotide 1004 and the auxiliary oligonucleotide 1006. The right-hand view of Fig. 10 shows the label 1000 along its longitudinal axis and schematically shows the duplex structure of the nucleic acid backbone 1002 as a double helix.

The labelling moieties 104 are attached to the main and auxiliary oligonucleotides 1004, 1006 such that they are evenly spaced around the duplex structure of the nucleic acid backbone 1002. For example, the evenly spaced attachment is achieved by selecting appropriate sequences for the main and auxiliary oligonucleotides 1004, 1006, which allow the controlled formation of the duplex.

In addition, or as alternative to specific sequences the main and auxiliary oligonucleotides 1004, 1006 may also comprise repetitive elements and/or may comprise poly(T), poly(A), poly(C), poly(G) stretches. In one preferred embodiment, the main oligonucleotide 1004 comprises poly(T) sequences, which can be manufactured in a particularly cost-effective manner. For example, a label may comprise poly(T) main oligonucleotide and the auxiliary oligonucleotide(s) may be one or multiple poly(A) oligonucleotides.

In another particularly preferred embodiment, the main and auxiliary oligonucleotides 1004, 1006 comprise specific sequences that allow the controlled hybridization, which enables precise control over labelling moieties 104 that are attached to both the main and auxiliary oligonucleotide. As a dsDNA helix makes a full-turn every 10nt, which corresponds roughly to 3.4 nm (referred to as the pitch), the attachment positions along the auxiliary and main oligonucleotides 1004, 1006 may be selected such that the positioning of labelling moieties 104 on the duplex is optimized with respect to dye-to-dye distance and orientation in space. This is schematically depicted in the right-hand view of Fig. 10.

In a particularly preferred embodiment, the dyes are conjugated to nucleobases to opposing nucleobases of the main and auxiliary oligonucleotides 1004, 1006 and the dye-to-dye distance along the main and auxiliary oligonucleotides 1004, 1006 is in the range of 3 nt to 30 nt, more preferably 3 nt to 20 nt.

In a particularly preferred embodiment, the main oligonucleotide 1004 of the label 1000 is attached to a first labelling moiety and the auxiliary oligonucleotide 1006 is attached to a second labelling moiety, wherein in the first and second labelling moieties comprise, or are, a FRET pair such as for example the following pairs of ATTO dyes:
FRET Donor - FRET Acceptor(s)
ATTO 425 - ATTO 520
ATTO 488 - ATTO 550, ATTO 565, ATTO 647**N**, ATTO 655
ATTO 520 - ATTO 647**N**
ATTO 532 - ATTO 647**N**, ATTO 655
ATTO 550 - ATTO 590, ATTO 647**N**
ATTO 565 - ATTO 590, ATTO 647**N**
ATTO 590 - ATTO 620, ATTO 647**N**, ATTO 680
ATTO 620 - ATTO 680

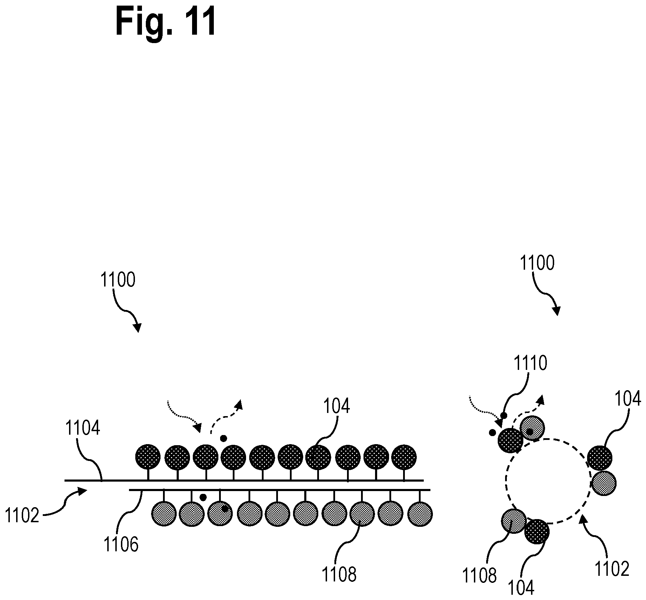

Figure 11 is a schematic view of a label 1100. The label 1100 comprises a nucleic acid backbone 1102 with a main oligonucleotide 1104 and an auxiliary oligonucleotide 1106. The main oligonucleotide 1104 and the auxiliary oligonucleotide 1106 form a duplex structure. A plurality of labelling moieties 104 is attached to the main oligonucleotide 1004, whereas a plurality of reactive oxygen species quenchers 1108 is attached to the auxiliary oligonucleotide 1006. The right-hand view of Fig. 11 shows the label 1100 along its longitudinal axis and schematically shows the duplex structure of the nucleic acid backbone 1102 as a double helix.

The reactive oxygen species quenchers 1108 are configured to reduce the occurrence or concentration of reactive oxygen species 1110 in their proximity. This enables an increase in the lifetime of the labelling moieties 104. Examples of the reactive oxygen species quenchers 1108 include DABCO and lycopene.

The labelling moieties 104 and the reactive oxygen species quenchers 1108 are attached to the respective main and auxiliary oligonucleotides 1104, 1106 such that each of the reactive oxygen species quenchers 1108 is in close proximity to one of the labelling moieties 104. Preferably, the reactive oxygen species quenchers may be attached to the nucleic acid backbone at a distance in a range of 0.3 nm to 15 nm to one of the labelling moieties.

A preferred arrangement for two labelling moieties such as a first labelling moiety 104 (e.g. dye) that shall be brought into close proximity to a second labelling moiety 1108 (e.g. ROS quencher or a FRET partner donor or acceptor) via the duplex formation between the main and auxiliary oligonucleotide 1004, 1006 is to place the first and second labelling moiety 104, 1108 at a distance of 3 to 6 nt along the duplex. On a natural dsDNA duplex 5 nt corresponds to a half helix turn, which means that if the first labelling moiety 104 is at position 1 on the main oligonucleotide 1104 and the second labelling moiety 1108 is placed at position 5 or 6 of the auxiliary oligonucleotide 1106 than the first and second labelling moieties 104, 1108 are on the same side of the helix and about 1.7 nm apart. In this case the positions are counted from the same end of the helix, i.e. base pairs share the same position count. In light of the fact that typical R0 values (Förster radii) for FRET between ATTO dye pairs (2-7 nm) as well as other organic fluorescent dyes, Qdots, Pdots, fluorescent proteins are typically in the range of 1-10 nm, this allows the effective generation of FRET-based labels. The attachment of FRET donor and acceptor to different, i.e. the main and auxiliary oligonucleotides 1104, 1106, is particular advantageous as it allows the effective generation of FRET-based labels from a library of conjugated main and auxiliary oligonucleotides 1104, 1106.

Further, the labelling moieties 104 and the reactive oxygen species quenchers 1108 may preferably be evenly spaced around the duplex structure of the nucleic acid backbone 1102.

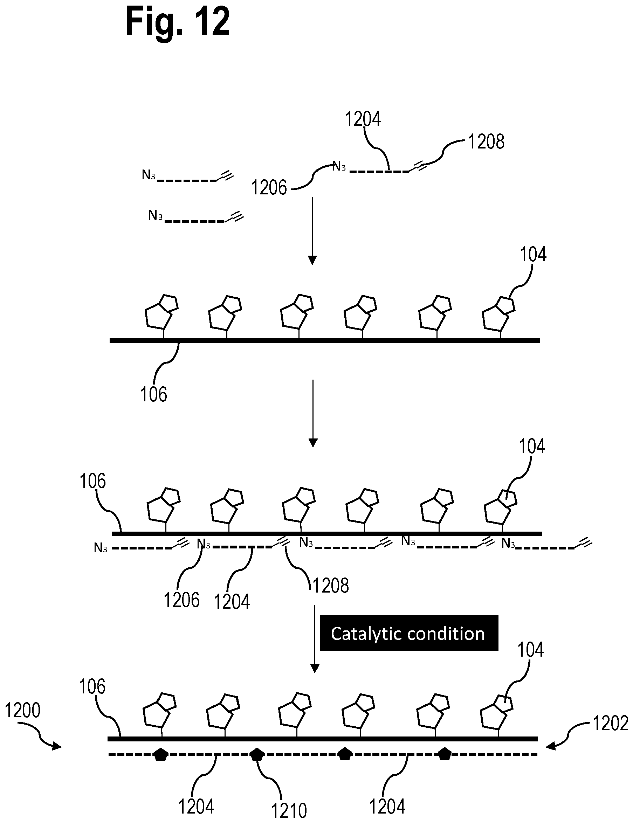

Figure 12 is a schematic view of a label 1200 and steps to generate the label 1200. The label 1200 comprises a nucleic acid backbone 1202 with the main oligonucleotide 106, to which labelling moieties 104 are attached. The nucleic acid backbone 1202 further comprises a plurality of auxiliary oligonucleotides 1204 hybridised to the main oligonucleotide 106. In order to hybridise the auxiliary oligonucleotides 1204 to the main oligonucleotide 106, the main oligonucleotide 106 may comprise a single continuous first nucleotide sequence and each of the auxiliary oligonucleotides 1204 may have a second sequence that is complementary to unique or repetitive, identical parts of the first nucleotide sequence of the main oligonucleotide 106. Alternatively, the main oligonucleotide 106 may comprise several unique or identical first nucleotide sequences that at least some of the auxiliary oligonucleotides 1204 are complementary to.

In addition, the auxiliary oligonucleotides 1204 comprise a first reaction moiety 1206 and a second reaction moiety 1208. In particular, the first reaction moiety 1206 is attached to one of a 3' and 5' end of the auxiliary oligonucleotide 1208 and the second reaction moiety 1208 is attached to the other one of the 3' and 5' end of the auxiliary oligonucleotide 1204.

The first reaction moiety 1206 and the second reaction moiety 1208 are configured to form a covalent bond 1210 with each other, in particular under specific catalytic conditions or in the presence of a particular catalyst. This cross-links the auxiliary oligonucleotides 1204 of the label 1200. For example, the first reaction moiety 1206 and the second reaction moiety 1208 may be respective click chemistry groups, in particular based on copper-catalysed azide-alkyne cycloaddition. This forms a triazole linkage between the auxiliary oligonucleotide 1204. In an alternative embodiment, the first reaction moiety 1206 and the second reaction moiety 1208 are configured to form a squaramide linkage. Generally, the covalent bond 1210 increases the stiffness of the nucleic acid backbone 1202 compared to phosphodiester bonds of natural nucleic acids.

The label 1200 may be generated by initially contacting main oligonucleotide 106 with the auxiliary oligonucleotides 1204 in order to hybridise the main oligonucleotide 106 with the auxiliary oligonucleotides 1204. This generates the duplex structure of the nucleic acid backbone 1202.

In a subsequent step, a particular catalytic condition may be applied to the auxiliary oligonucleotides 1204, such as the addition of a catalyst. This forms the covalent bond 1210 between the auxiliary oligonucleotides 1204.

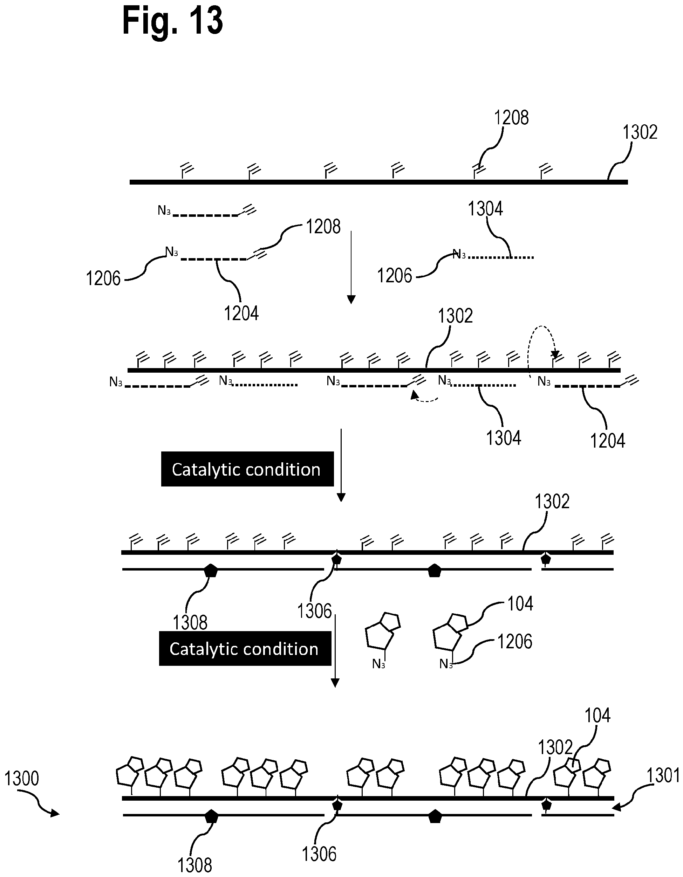

Figure 13 is a schematic view of a label 1300 and steps to generate the label 1300. The label 1300 comprises a nucleic acid backbone 1301 with a main oligonucleotide 1302 and a plurality of auxiliary oligonucleotides 1204, 1304. The main oligonucleotide 1302 comprises a first nucleotide sequence that the second nucleotide sequence of each auxiliary oligonucleotide 1204, 1304 are complementary to. In particular, the auxiliary oligonucleotides 1204 may have a different second nucleotide sequence than the auxiliary oligonucleotide 1304. This enables generating the label 1300 with a particular order of the auxiliary oligonucleotides 1204, 1304 along the duplex structure of the nucleic acid backbone 1301.

The auxiliary oligonucleotides 1204 comprise the first reaction moiety 1206 and the second reaction moiety 1208. The auxiliary oligonucleotides 1304 comprise only the first reaction moiety 1206, preferably at a 3' or 5' end. The main oligonucleotide 1302 comprises several second reaction moieties 1208.

The second reaction moieties 1208 are arranged along the main oligonucleotide 1302 such that they are at a distance from the first reaction moieties 1206, when the second nucleotide sequence of the auxiliary oligonucleotides 1304 are hybridised to the first nucleotide sequence of the main oligonucleotide 1302, that respective first and second reaction moieties 1206, 1208 can form a covalent bond 1306.

The first reaction moieties 1206 of the auxiliary oligonucleotides 1304 may form a covalent bond 1308 with second reaction moieties 1208 of adjacent auxiliary oligonucleotides 1204.

Generally, the covalent bonds 1306, 1308 cross-link the nucleic acid backbone 1301 and increase the stiffness of the nucleic acid backbone 1301 compared to base-pairing interactions or phosphodiester bonds of natural nucleic acids.

Optionally, the labelling moieties 104 may similarly comprise first reaction moieties 1206 and be attached to respective second reaction moieties 1208 of the main oligonucleotide 1302.

The label 1300 may be generated by initially hybridising the auxiliary oligonucleotides 1304, 1204 to the main oligonucleotide 1302. Subsequently, the auxiliary oligonucleotides 1304, 1204 and the main oligonucleotide 1302 may be cross-linked by applying a catalytic condition to form covalent bonds 1306, 1308 between the auxiliary oligonucleotides 1304, 1204 and the main oligonucleotide 1302.

In a subsequent step, the labelling moieties 104 with first reaction moieties 1206 may be brought into contact with the main oligonucleotide 1302 and the labelling moieties 104 may be attached to the main oligonucleotide 1302 by applying a respective catalytic condition.

In a particular embodiment, the first and second reaction moieties 1206, 1208 for attaching the labelling moieties 104 to the main oligonucleotide 1302 may be different to the first and second reaction moieties 1206, 1208 for cross-linking the auxiliary oligonucleotides 1304, 1204 and the main oligonucleotide 1302.

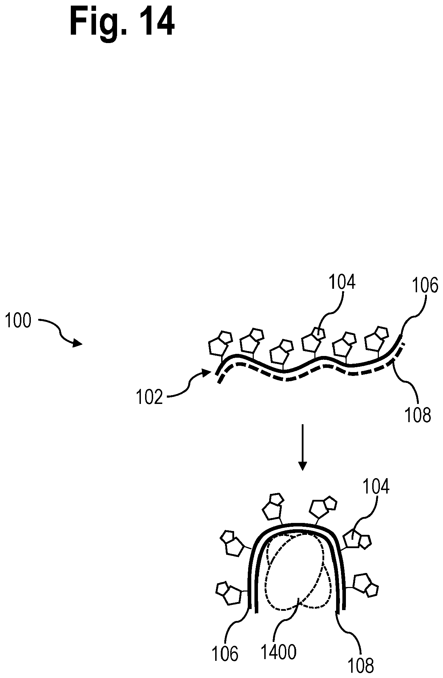

Figure 14 is a schematic view of a label 100 and a complex of the label 100 with a nucleic acid binding molecule 1400. The nucleic acid backbone 102 of the label 100 may be bound to the nucleic acid binding molecule 1400 in order to bend the backbone 102 and increase the stiffness or rigidity of the nucleic acid backbone 102.

The nucleic acid binding molecule 1400 may be a transcription factor (e.g. bHLH), a histone, or prokaryotic histones such as IHF or HU. Some nucleic acid binding molecules may bind nucleic acid duplex structures in a sequence-specific manner, others in an unspecific manner. Depending on the particular nucleic acid binding molecule 1400, the nucleic acid backbone 102, in particular its duplex structure, may comprise a particular recognition sequence.

An example of a nucleic acid binding molecule is disclosed by Rice et al. Cell, Vol. 87, 1295-1306, December 27, 1996 (Crystal Structure of an IHF-DNA Complex: A Protein-Induced DNA U-Turn). Using IHF as a nucleic acid binding molecule may bend the nucleic acid backbone 102 into one or a plurality of U-turns. This allows for label compaction and labelling moiety placing on the backbone 102 in a way that achieves optimal spatial separation of the labelling moieties 104.

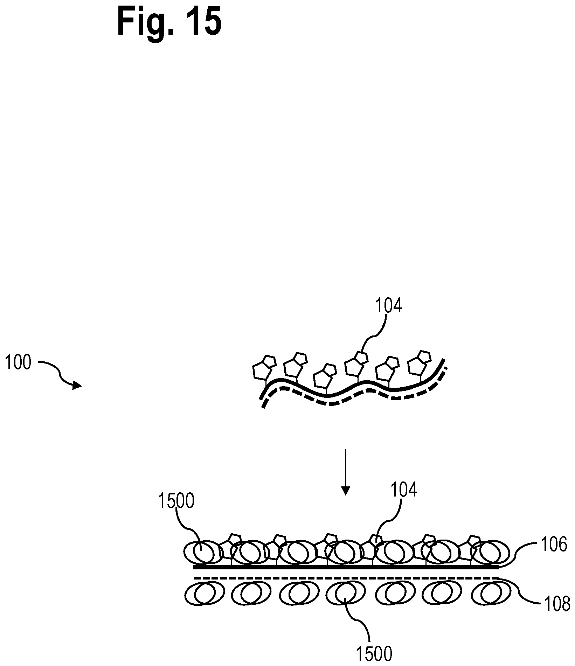

Figure 15 is schematic view of the label 100 and a complex of the label 100 with a nucleic acid binding molecule 1500. The nucleic acid backbone 102 of the label 100 may be bound to the nucleic acid binding molecule 1500 in order to increase the stiffness or rigidity of the nucleic acid backbone 102.

Suitable nucleic acid binding molecules may include histones. Histones with unconventional DNA-binding modes in vitro are major chromatin constituents in the bacterium Bdellovibrio bacteriovorus (Hocher et al., Nature Microbiology, Volume 8, November 2023, 2006-2019).

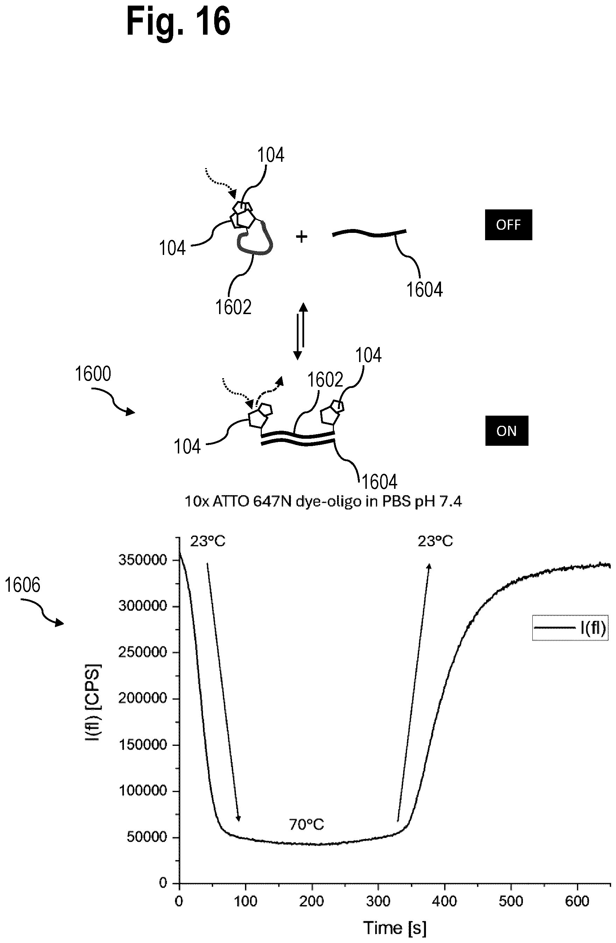

Figure 16 is a schematic view of steps to control optical properties of a label 1600. The label 1600 comprises a labelling moiety 104, in particular with a strong tendency for aggregation, such as a hydrophobic labelling moiety. The label 1600 further comprises a duplex structure forming nucleic acid backbone with a main oligonucleotide 1602 and an auxiliary oligonucleotide 1604.

The fluorescence emission of the labelling moieties 104 of the label 1600 may be controlled by initially increasing the temperature, in particular above the melting temperature of the duplex structure of the nucleic acid backbone, and thereby removing the auxiliary oligonucleotide 1604. This results in the main oligonucleotide 1602 having an increased flexibility and enables the labelling moieties 104 to aggregate. The aggregated labelling moieties 104 have a drastically reduced fluorescence emission, for example due to self-quenching.

A subsequent decrease in temperature results in the auxiliary oligonucleotide 1604 again hybridising to the main oligonucleotide 1602 and formation of the duplex structure of the nucleic acid backbone of the label 1600. Thus, the fluorescence emission of the label 1600 can be switched on and off by a change in temperature.

The label 1600 may be introduced into a biological sample prior to carrying out the steps of increasing and subsequently decreasing the temperature. The label 1600 may be attached to an affinity reagent (not shown) in order to specifically bind the label 1600 to a target analyte in the biological sample.

The graph 1606 shows experimental data of the effect described above. The graph showing fluorescent emission of a label with ten ATTO 647N fluorophores over time. In particular, the label used has a the length of the nucleic acid backbone of about 120 nt and the dye-to-dye spacing is 8 nt and the label comprises a dsDNA nucleic acid backbone comprising a first oligonucleotide 1602 and a second oligonucleotide 1604. The graph 1606 shows a time course, wherein the temperature of the label is changed from 23°C to 70°C (between about 0 and 50 s) and then from 70°C back to 23°C (between about 350 to 400 s). As a result of melting the duplex the nucleic acid backbone of said label is changed from a dsDNA backbone to a ssDNA backbone resulting in higher flexibility and dye aggregation, which leads to a reduction in fluorescence emission (between about 50 to 350 s). Following to the cooling the duplex is formed again by reannealing the oligonucleotides 1602 and 1604 and hence the dsDNA nucleic acid backbone is formed again and the fluorescence emission increases, as the dsDNA backbone's higher rigidity reduces the dye aggregation.

Identical or similarly acting elements are designated with the same reference signs in all Figures. As used herein the term "and/or" includes any and all combinations of one or more of the associated listed items and may be abbreviated as "/".

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

### Reference signs

100, 200, 500, 702, 802, 900, 901, 1000, 1200, 1300, 1600 Label
102, 202, 502, 1002, 1102, 1202, 1301 Nucleic acid backbone
104 Labelling moiety
106, 504, 902, 910, 1004, 1104, 1302, 1602 Main oligonucleotide
108, 906, 912, 1006, 1106, 1204, 1304, 1604 Auxiliary oligonucleotide
202 Flexible nucleic acid backbone
506 First nucleotide sequence
508 Second nucleotide sequence
600 Double helix
700,800 Marker
704 Affinity reagent
706 Barcode oligonucleotide
708 Attachment oligonucleotide
710 Free end of main oligonucleotide
712 Second free end of main oligonucleotide
804 Complementary barcode oligonucleotide
806 Further end of main oligonucleotide
904, 914, 1206 First reaction moiety
908, 916, 1208 Second reaction moiety
918, 1210, 1306, 1308 Covalent bond
920 Free end of main oligonucleotide
1108 Reactive oxygen species quencher
1110 Reactive oxygen species
1400, 1500 Nucleic acid binding molecule
1606 Graph

## Claims

1. A label (100, 200, 500, 702, 802, 900, 901, 1000, 1200, 1300, 1600) for analysing a biological sample comprising
a nucleic acid backbone (102, 502, 1002, 1102, 1202, 1301),
a first plurality of labelling moieties (104), and
wherein the nucleic acid backbone (102, 502, 1002, 1102, 1202, 1301) has a duplex structure comprising at least 50% of nucleotides of the nucleic acid backbone (102, 502, 1002, 1102, 1202, 1301).

2. The label according to claim 1, wherein the nucleic acid backbone (102, 502, 1002, 1102, 1202, 1301) comprises a main oligonucleotide (106, 504, 902, 910, 1004, 1104, 1302, 1602) with at least a first nucleotide sequence (506), wherein the nucleic acid backbone (102, 502, 1002, 1102, 1202, 1301) comprises at least a second nucleotide sequence (508), and wherein at least the first nucleotide sequence (506) and the second nucleotide sequence (508) form the duplex structure of the nucleic acid backbone (102, 502, 1002, 1102, 1202, 1301).

3. The label according to claim 2, wherein the main oligonucleotide (106, 504, 902, 910, 1004, 1104, 1302, 1602) further comprises the second nucleotide sequence (508), and wherein the second nucleotide sequence (508) is a reverse complement of the first nucleotide sequence (506).

4. The label according to claim 3, wherein the nucleic acid backbone (102, 502, 1002, 1102, 1202, 1301) comprises several of the main oligonucleotide (106, 504, 902, 910, 1004, 1104, 1302, 1602).

5. The label according to one of the preceding claims 2 to 4, wherein the nucleic acid backbone (102, 502, 1002, 1102, 1202, 1301) comprises at least one auxiliary oligonucleotide (108, 906, 912, 1006, 1106, 1204, 1304, 1604) comprising the second nucleotide sequence (508).

6. The label according to claim 5, wherein the main oligonucleotide (106, 504, 902, 910, 1004, 1104, 1302, 1602) and the auxiliary oligonucleotide (108, 906, 912, 1006, 1106, 1204, 1304, 1604) are cross-linked with each other.

7. The label according to one of the preceding claims 2 to 6, wherein the nucleic acid backbone (102, 502, 1002, 1102, 1202, 1301) comprises a plurality of the first nucleotide sequence (506).

8. The label according to one of the preceding claims, wherein the duplex structure only comprises adenine and thymine nucleotides.

9. The label according to one of the preceding claims, comprising at least one nucleic acid binding molecule (1400, 1500).

10. The label according to one of the preceding claims, wherein the labelling moieties (104) are hydrophobic labelling moieties.

11. The label according to one of the preceding claims, wherein the labelling moieties (104) are attached to the nucleic acid backbone (102, 502, 1002, 1102, 1202, 1301) at a distance from each other in a range from one to ten nucleotides.

12. The label according to one of the preceding claims, wherein the labelling moieties (104) are attached to the duplex structure.

13. The label according to one of the preceding claims, wherein the auxiliary oligonucleotide (108, 906, 912, 1006, 1106, 1204, 1304, 1604) comprises at least a second plurality of labelling moieties configured to excitonically interact with the first plurality of labelling moieties (104).

14. The label according to one of the preceding claims, comprising a plurality of reactive oxygen species quenchers (1108), wherein each of the reactive oxygen species quenchers (1108) is attached to the nucleic acid backbone (102, 502, 1002, 1102, 1202, 1301) in close proximity to a labelling moiety (104).

15. The label according to one of the preceding claims, wherein the nucleic acid backbone (102, 502, 1002, 1102, 1202, 1301) comprises 20 to 200 nucleotides and/or wherein the main oligonucleotide (106, 504, 902, 910, 1004, 1104, 1302, 1602) has a length of 20 to 120 nucleotides.

16. The label according to one of the preceding claims, wherein the nucleic acid backbone (102, 502, 1002, 1102, 1202, 1301), in particular the main oligonucleotide (106, 504, 902, 910, 1004, 1104, 1302, 1602), comprises a barcode sequence, wherein the barcode sequence preferably is configured to bind the label to a complementary barcode sequence of an affinity reagent and thereby allow attaching the label to a barcoded affinity reagent.

17. The label according to any of the preceding claims comprising at least 5 labelling moieties, in particular, the labelling moieties are covalently conjugated to either the main oligonucleotide or to the auxiliary oligonucleotide.

18. The label according to any of the preceding claims, wherein the nucleic acid backbone comprises nucleic acid analogues, in particular modified nucleobases.

19. A marker (700, 800) for analysing a biological sample comprising:
a label (100, 200, 500, 702, 802, 900, 901, 1000, 1200, 1300, 1600) according to one of the preceding claims, and
an affinity reagent (704) configured to specifically bind to a target analyte.

20. The marker according to claim 19, wherein the affinity reagent comprises a barcode sequence (804).

21. A method for analysing a biological sample comprising the steps:
introducing into the biological sample at least one marker or parts of at least one marker (700, 800) according to one of the preceding claims 19 and 20, and
generating a readout of the biological sample with the marker (700, 800).
